# EUROPEAN PATENT APPLICATION

(11) **EP 3 915 366 A1**
(43) Date of publication of application: **01.12.2021**
(21) Application number: 20741731.2
(22) Date of filing: 08.01.2020
(51) Int. Cl.: A01N 1/02, C07K 7/64, C07K 7/56, A61K 38/13, A61K 38/12

(54) **CYCLOSPORIN ANALOG AND USE THEREOF**

(30) Priority: 20.01.2019 CN 201910050741
(71) Applicant: Farsight Medical Technology (Shanghai) Co., Ltd., Shanghai 201210 (CN)
(72) Inventor: MAK, Ching Pong, Shanghai 201210 (CN); ZENG, Li, Shanghai 201210 (CN); YU, Shengqiang, Shanghai 201210 (CN); PEEL, Michael Robert, Cambridgeshire CB22 3AT (GB); FLIRI, Hans Georg, Cambridgeshire CB22 3AT (GB)
(74) Representative: Pharma Patents International AG
(86) International application number: PCT/CN2020/070852
(87) International publication number: WO 2020/147624

(57) **Abstract**

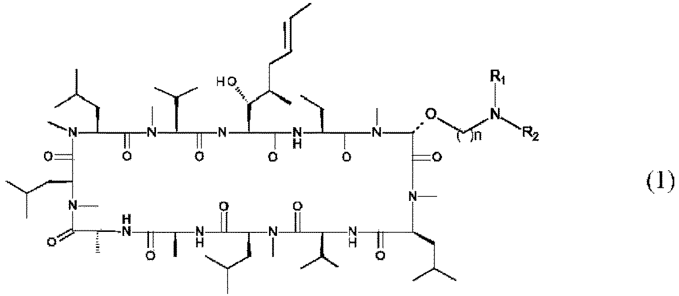

The present invention provides a cyclosporin analog and use thereof, and in particular relates to a compound and use thereof as a mitochondrial protective agent for storing a donated organ. The compound is a compound of formula 1 or a salt thereof, wherein n is 2-5, and R₁ and R₂ are independently selected from H or C₁-C₄ alkyl, wherein R₁ and R₂ can be linked together to form a C₃-C₅ heteroalkyl ring.

## Description

### Field of the invention

The present invention relates to cyclosporin analogues and their use as mitochondrial protectants in organ donors. The compounds of the invention may be used for the preservation of organs or other body parts removed from or severed from a subject prior to transplantation. In particular, though not exclusively, the invention relates to the use of a cyclosporin analogue of Formula 1 as a mitochondrial protectant in an organ donor. More particularly, the invention relates to the use of Compound 1 as a mitochondrial protectant in a kidney donor.

### Background art

Acute inflammation is well recognized to participate in the complex interaction of various cellular (neutrophils, macrophages) and extracellular (complement, histamine) factors that act in response to PAMP (pathogen-activated molecular patterns) and DAMP (damage-activated molecular patterns) signals to resolve the originating insult. Cyclophilin A has been demonstrated to function as a chemokine to facilitate leukocyte migration in support of an inflammatory response and blockade of cyclophilin A was shown to be beneficial in animal models of acute inflammation. More recently a severe form of inflammation that is accompanied by cell death and tissue necrosis has been described. A significant body of evidence now supports the opening of a pore at the mitochondrial membrane, termed the Mitochondrial Permeability Transition Pore (MPTP), as being critical to the onset and maintenance of this necrotic inflammation. A key regulator of this MPTP opening is Cyclophilin D (CypD), and inhibitors of CypD have shown good activity in preventing tissue damage associated with necrotic inflammation. Opening of the MPTP, and subsequent initiation of necrotic cell death, is triggered by elevated intracellular calcium levels that result from a variety of factors including oxidative stress, hypoxia, bile salt toxins, etc. Notably, genetic ablation, or pharmacological inhibition, of CypD was found to be protective toward tissue degradation due to ischemia-reperfusion injury of cardiac tissue suggesting that CypD inhibition is a viable drug target for ischemia-reperfusion injury more generally.

Renal ischemia results from arterial occlusion, shock and kidney transplantation and can lead to renal cell death and kidney failure. A further source of tissue damage associated with ischemia occurs during the process of organ transplantation. Following removal of the donor organ the tissue is inevitably subject to oxygen starvation as a result of loss of blood flow, and damage to the ischemic tissue ensues upon re-initiation of flow. A compound that is able to prevent tissue damage during the resection and reperfusion process would improve the viability of the transplanted organ. A preferred profile for a compound to be used as a tissue protectant would include potent inhibition of CypD, prevention of MPTP opening following ischemic stress, and solubility sufficient to be formulated for intravenous administration and for adding to the preservation solutions typically used during organ transportation, in concentrations high enough to protect the tissue.

In studies carried out using cyclophilin D knockout mice as well as pharmacological strategies with cyclophilin inhibitors it has been unambiguously demonstrated that the mitochondrial permeability transition pore (MPTP), a non-specific channel in the inner mitochondrial membrane, is a fundamental event in cell death that results from a variety of insults. Further, inhibition of cyclophilin D can prevent opening of the mPTP which is protective toward mitochondrial function and preserves cell viability. Toxic insults to the cell which can induce MPTP include ischemia, Reactive Oxygen Species (ROS), bile salts, oligomers of alpha-synuclein, and elevated intracellular calcium levels. Donated organs after removal from the donor can undergo necrotic inflammation resulting in tissue damage and impaired function when placed into a recipient. Compounds described herein prevent the degradation of the donated organ after removal whilst the organ is stored waiting for implantation to the recipient.

Cyclosporin A is a compound well known for its immunosuppressive properties, but other biological properties have also been described. Cyclosporin A has the following chemical structure:

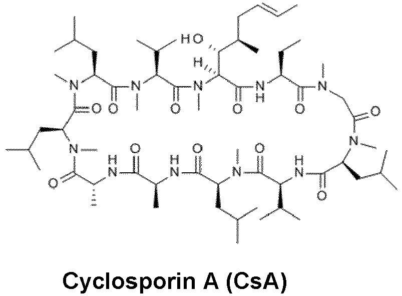

Biologically active derivatives of Cyclosporin A have also been made. For example, US 6,583,265, EP 0 484 281 and EP 0 194 972 describe cyclosporin derivatives having various properties including immunosuppressive, antiparasitic and antiviral properties.

US 6,583,265 describes cyclosporin derivatives with modifications made at position 3 of the cyclosporin macrocycle. In particular, US 6,583,265 discloses Compound 1:

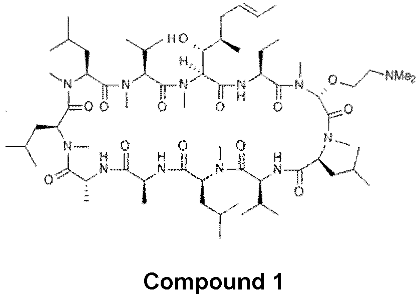

This compound is example 27 in the patent US 6,583,265, which includes many hundreds of named compounds having modifications at various positions around the ring. However no biological testing data or particular uses are described for this compound or related analogues. When the applicants tried to synthesise said compound, using the published route used to prepare Compound 27 in US 6,583,265, the method was not effective. Many attempts were made to replicate the methodology in US 6,583,265 without great success. Without being bound by theory, it is believed that the dimethyl amino group (being basic) reacted preferentially with the acid catalyst. The acid catalyst was thereby prevented from activating the loss of the leaving group, inhibiting the progress of the reaction. There is thus some doubt regarding whether Example 27 was previously synthesised, and therefore doubts as to whether this prior art is actually an enabling disclosure for the preparation of Compound 1.

### Statements of invention

Applicants have identified that compounds which act as mitochondrial protectants can be given to organ donors in order to improve preservation of the organs pre-implantation. The compounds act via cyclophilin D inhibition. Any known inhibitor of Cyclophilin D may be used as described herein. Suitable inhibitors include cyclosporin or cyclic depsipeptide analogues such as those reported in publications including for example, US 6,583,265, EP 0 484 281, EP 0 194 972, WO2010/076329 and WO2014/053834.

Applicants have synthesised compounds previously suggested in the prior art publications above and surprisingly discovered that a small subset of the compounds are particularly good Cyclophilin D inhibitors. Thus the compounds described below may be used in the treatment of conditions benefiting from inhibitions of Cyclophilin D activity, and may be administered to organ donors to aid preservation of the organ ex-vivo. According to an aspect of the invention, there is provided a compound for use as a mitochondrial protectant in the preservation of a removed body part or organ from a donor prior to transplantation. The removed body part may be a limb, hand, foot, finger or toe. The organ may be a kidney. The donor may be a live donor. 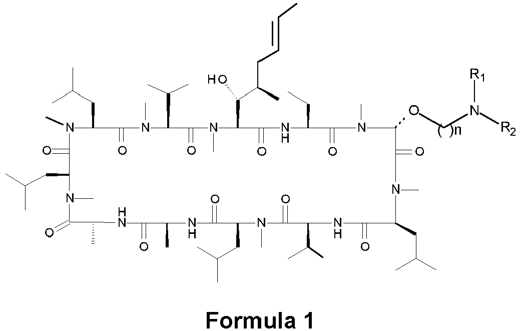 or a salt thereof,
wherein n is 2-5, and
R₁ and R₂ are independently selected from H or C₁-C₄ alkyl, wherein R₁ and R₂ may be joined together to form a C₃-C₅ heteroalkyl ring.

In a preferred embodiment, the compound is:

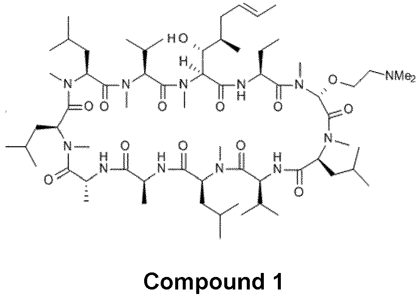

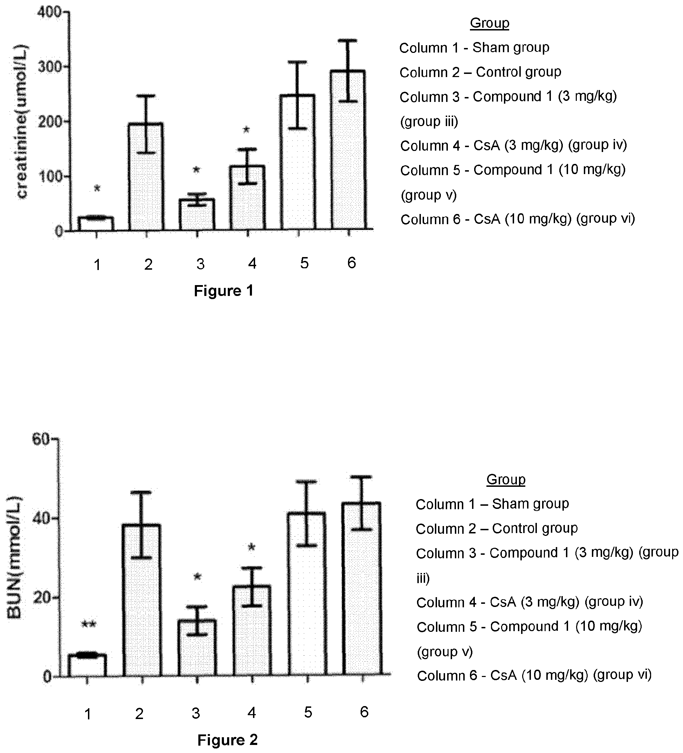

The compound of Formula 1 can be used in the treatment of ischaemia-reperfusion injury associated with the re-attachment of severed body parts and organs. The data provided with this application, shown graphically in Figures 1 and 2, shows that Compound 1 significantly outperformed the well-established comparative example Cyclosporin A and other closely related analogues, including those within the scope of Formula 1. Indeed in the challenging test performed, where blood was deprived from an organ for thirty minutes, Compound 1 gave surprisingly good results, indeed showing a near complete reversal in the expected damage to the affected organs.

Compound 1 shows a remarkable potency as an inhibitor of Cyclophilin D. As seen in table 1, compound 1 (entry 4) shows an EC₅₀ of 24 nM against cyclophilin D. Replacement of one methyl group on the N atom with H (entry 1 is simply NHMe ve NMe₂) reduces the potency by approximately 100-fold, with an EC₅₀ of >2000 nM. Thus compound 1 is a surprisingly potent inhibitor of cyclophilin D and Mitochondrial Permeability Transition (MPT).

In an embodiment, of the compound, method or use as mentioned herein above, the dose of the compound is 0.1 to 10 mg/kg. In an embodiment, of the compound, method or use as mentioned herein above, the dose of the compound is 1 to 3 mg/kg. In these dose ranges the compound of the invention is especially effective.

Salts of the invention may result from the addition of acids to the compound of Formula 1, or Compound 1. The resultant acid addition salts include those formed with acetic, 2,2-dichloroacetic, citric, lactic, mandelic, glycolic, adipic, alginic, aryl sulfonic acids (e.g., benzenesulfonic, naphthalene-2-sulfonic, naphthalene-1,5-disulfonic and p-toluenesulfonic), ascorbic (e.g. L-ascorbic), L-aspartic, benzoic, 4-acetamidobenzoic, butanoic, (+) camphoric, camphor-sulfonic, (+)-(1S)-camphor-10-sulfonic, capric, caproic, caprylic, cinnamic, citric, cyclamic, dodecylsulfuric, ethane-1,2-disulfonic, ethanesulfonic, 2-hydroxyethanesulfonic, formic, fumaric, galactaric, gentisic, glucoheptonic, gluconic (e.g. D-gluconic), glucuronic (e.g. D-glucuronic), glutamic (e.g. L-glutamic)], α-oxoglutaric, glycolic, hippuric, hydrobromic, hydrochloric, hydriodic, isethionic, lactic (e.g. (+)-L-lactic and (±)-DL-lactic), lactobionic, maleic, malic (e.g. (-)-L-malic), (±)-DL-mandelic, metaphosphoric, methanesulfonic, 1-hydroxy-2-naphthoic, nicotinic, nitric, oleic, orotic, oxalic, palmitic, pamoic, phosphoric, propionic, L-pyroglutamic, salicylic, 4-amino-salicylic, sebacic, stearic, succinic, sulfuric, tannic, tartaric (e.g.(+)-L-tartaric), thiocyanic, undecylenic and valeric acids. In particular acid addition salts include those derived from mineral acids such as hydrochloric, hydrobromic, phosphoric, metaphosphoric, nitric and sulfuric acids; from organic acids, such as tartaric, acetic, citric, malic, lactic, fumaric, benzoic, glycolic, gluconic, succinic, arylsulfonic acids.

The compound of the invention may be administered together with one or more further active substances.

According to one aspect of the invention, there is provided a mitochondrial protectant compound for use in preserving a body part or organ removed from or severed from a subject and prior to organ transplantation to a new individual or re-attachment of the body part, wherein the compound is a compound of Formula 1: 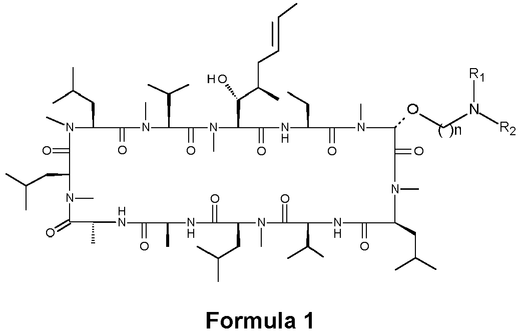 or a salt thereof,
wherein n is 2-5, and
R₁ and R₂ are independently selected from H or C₁-C₄ alkyl, wherein R₁ and R₂ may be joined together to form a C₃-C₅ heteroalkyl ring.

According to one aspect of the disclosure, there is provided a method for preserving an organ removed from or severed from a subject and prior to organ transplantation or re-attachment, comprising exposing the organ to a mitochondrial protectant compound of Formula 1: 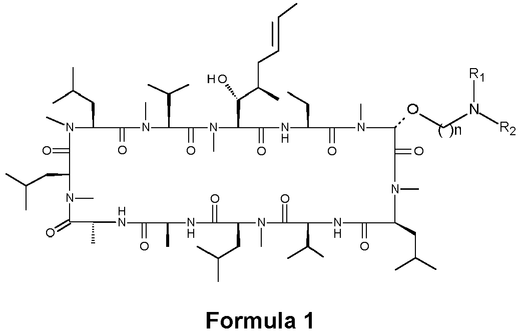 or a salt thereof,
wherein n is 2-5, and
R₁ and R₂ are independently selected from H or C₁-C₄ alkyl, wherein R₁ and R₂ may be joined together to form a C₃-C₅ heteroalkyl ring.

According to one aspect of the disclosure, there is provided a use of a mitochondrial protectant compound for the manufacture of a medicament for the preservation of a body part or organ removed from or severed from a subject and prior to transplantation or re-attachment, wherein the compound is a compound of Formula 1: 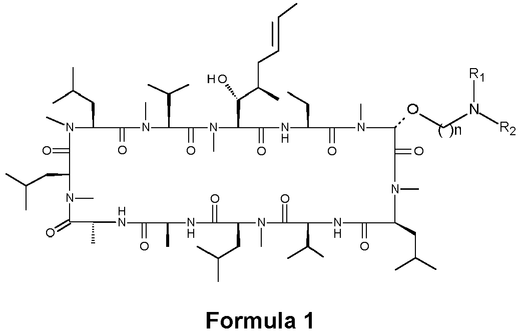 or a salt thereof,
wherein n is 2-5, and
R₁ and R₂ are independently selected from H or C₁-C₄ alkyl, wherein R₁ and R₂ may be joined together to form a C₃-C₅ heteroalkyl ring.

According to one aspect of the disclosure, there is provided a use of a mitochondrial protectant compound for the preservation of a body part or organ removed from or severed from a subject and prior to transplantation or re-attachment, wherein the compound is a compound of Formula 1: 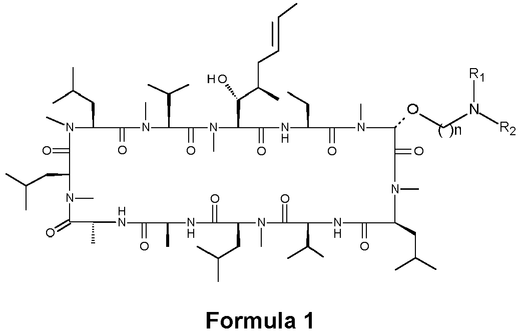 or a salt thereof,
wherein n is 2-5, and
R₁ and R₂ are independently selected from H or C₁-C₄ alkyl, wherein R₁ and R₂ may be joined together to form a C₃-C₅ heteroalkyl ring.

In an embodiment a compound may be used as a mitochondrial protectant in an organ donor, wherein the compound is administered to the organ donor in order to protect the organ prior to removal of said organ from said donor.

In a further embodiment the mitochondrial protectant compound is a cyclophilin inhibitor.

In an embodiment a compound may be used as a mitochondrial protectant in an organ donor, wherein the compound is administered to the organ donor in order to protect the organ prior to removal of said organ from said donor, wherein the compound is a compound of Formula 1: 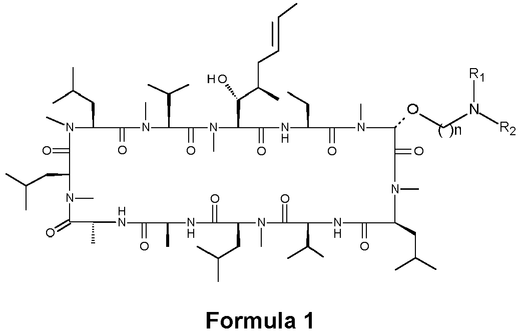 or a salt thereof,
wherein n is 2-5, and
R₁ and R₂ are independently selected from H or C₁-C₄ alkyl, wherein R₁ and R₂ may be joined together to form a C₃-C₅ heteroalkyl ring.

In an embodiment the compound of Formula 1 may be used as a mitochondrial protectant in an organ donor, wherein the organ is a kidney.

In an embodiment the compound of Formula 1 may be used for preserving a kidney. According to another aspect of the invention, there is provided a method of preserving an organ in an organ donor comprising administering a mitochondrial protectant compound to said donor prior to removal of said organ from said donor.

In an embodiment, there is provided a method of preserving a kidney in a kidney donor comprising administering a mitochondrial protectant compound to said donor prior to removal of said kidney from said donor.

In an embodiment, there is provided a method of preserving a kidney in a kidney donor comprising administering a mitochondrial protectant compound to said donor prior to removal of said kidney from said donor, wherein the compound is a compound of Formula 1.

In an embodiment, the compound of Formula 1 is administered to a live donor prior to organ transplantation.

In a preferred embodiment, the compound of Formula 1 is administered to a live kidney donor prior to kidney transplantation.

In an embodiment, there is provided a method of preserving a kidney in a kidney donor comprising administering a mitochondrial protectant compound to said donor prior to removal of said kidney from said donor, wherein the compound is Compound 1:

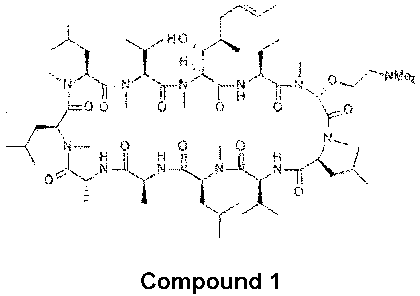

In an embodiment a compound may be used as a mitochondrial protectant in an organ donor, wherein the compound is administered to the organ donor in order to protect the organ prior to removal of said organ from said donor, wherein the compound is Compound 1.

In a preferred embodiment, Compound 1 may be used for preserving a kidney.

In an embodiment, there is provided a method of preserving a kidney in a kidney donor comprising administering a mitochondrial protectant compound to said donor prior to removal of said kidney from said donor, wherein the compound is Compound 1. In a preferred embodiment, Compound 1 is administered to a live kidney donor prior to kidney transplantation.

According to one aspect of the invention, there is provided a use of a mitochondrial protectant compound for the manufacture of a medicament for the preservation of a kidney. In an embodiment, there is provided a use of a mitochondrial protectant compound for the manufacture of a medicament for the preservation of a kidney, wherein the compound is a compound of Formula 1. In an embodiment, there is provided a use of a mitochondrial protectant compound for the manufacture of a medicament for the preservation of a kidney, wherein the compound is Compound 1.

According to an aspect of the invention, there is provided a method of preserving a kidney comprising treating a donor with Compound 1. In a preferred emodiment the organ or kidney donor is a live donor.

Ischemic injury occurs when the blood supply to an area of tissue is cut off. The incidence of ischemic injury is vast: myocardial infarction, stroke, and other thrombotic events and these affect more than 1.3 million individuals each year in the USA alone. In addition, ischemic injury also occurs during surgery when blood vessels are cross-clamped, and in organs for transplantation. The length of time a tissue can survive oxygen deprivation varies, but eventually ischemic tissue becomes necrotic.

Reperfusion (reoxygenation) injury is the tissue damage caused when the blood supply returns to the tissue after a period of ischemia or lack of oxygen (anoxia, hypoxia). Without being bound by theory, it is believed that the absence of oxygen and nutrients from the blood during the ischemic period creates a condition in which the restoration of circulation results in inflammation and oxidative damage.

In organ transplantation, there is a period of time between removing an organ from the donor's blood supply until the reconnection of the organ to the donor recipient's blood supply. During this period there is the potential for ischaemia-reperfusion injury. In some cases, organs may need to be transported long distances to the location of surgery, increasing the likelihood of organ damage.

In accidents involving severed limbs, there is a period of time between the severing of the body part from the blood supply until the reconnection of the body part to the blood supply. During this period there is the potential for ischaemia-reperfusion injury. In some cases, body parts and patients may need to be transported long distances to the location of surgery, increasing the likelihood of damage before, during and after re-attachment.

The invention is provided for administration of the mitochondrial protectant compounds of the invention to prevent this damage to body parts and organs. In particular in the period of time between removing a body part or organ from the donor's blood supply to reconnection to the donor recipient's blood supply or in the case of severed body parts, reattachment. The skilled person will be aware of ways in which the compounds of the invention can be administered to an individual prior to removal of a body part or organ or to a body part or organ removed from an individual, be they an organ donor or accident victim. For example, the compound of the invention could be administered intravenously to a donor or accident victim prior to removal of a body part or organ or could be added to (or included in) a fluid in which the organ is placed; and/or the compound of the invention could be added to (or included in) a fluid that is recirculated in or through the organ/body part.

In an embodiment, of the compound, method or use as mentioned herein above, the compound of the invention is administered to the organ after the removal of the organ from the individual and prior to transplantation or re-attachment. Alternatively, or in addition, the compound of the invention is administered to the donor subject prior to removal of the donor organ. For example, the compound of the invention may be administered systemically. An injection is one way to administer a systemic dose of the compound of the invention. The compound of the invention may also be administered to the recipient after organ transplantation or to the accident victim after re-attachment.

A systemic dose of the compound of the invention can be administered to the organ donor prior to organ removal. This allows for the organ to receive a protective dose of the compound prior to removal, thereby preserving the organ by protecting the organ from damage during the removal, and up to and during the process of transplantation into the donor recipient. In the case where more than one organ is being removed from a donor, this systemic dose ensures each organ receives a dose of the compound. A systemic dose is also more likely to provide an even dose of the compound to the organ tissue that is to be transplanted. In the case where the donor is legally dead, the dose can be greater than would normally be given to a living subject.

The compound can be administered shortly before organ removal surgery, or during organ removal surgery. For example, the compound of the invention may be administered 8, 7, 6, 5, 4, 3, 2 or 1 hour(s) before surgery.

In addition, or in the alternative, the organ recipient or accident victim may receive a dose of the compound of the invention prior to receiving the organ or undergoing re-attachment surgery, such that their blood supply contains a protective dose of the compound of the invention, thereby preserving the transplanted or re-attached body part from damage after surgery.

The body part may be severed from and re-attached to the same individual, or may be given to a second individual as a transplant. Where the body part is severed from a subject, the severing may be complete or partial. Partial severing may be for example severing of the blood supply but the body part remaining attached for example via skin, bone or muscle tissue. The compound may administered (i) to a severed body part; and/or (ii) to the subject prior to reattachment of the body part; and/or (iii) to the subject during or after re-attachment of the body part.

The invention also has application in non-human subjects e.g. cats, dogs, horses and pigs. The invention also has application in transgenic animals (e.g. transgenic pigs), where such animals have organs suitable for human transplantation.

In an embodiment, of the compound, method or use as mentioned herein above, the compound is Compound 1:

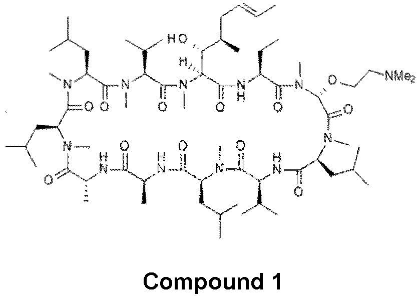

Compound 1 has been shown in this application to be an effective mitochondrial protectant.

The results displayed in Table 1 demonstrate the unexpectedly high Cyclophilin D inhibition and MPT of Compound 1 (entry 4) relative to similar analogues known in the art (entries 1-3 and 5-7). A 100 fold improvement in MPT was observed relative to three other closely related compounds (entries 1, 5 and 7) and an over 25 fold improvement was observed relative to the next best performing analogue (entry 3). Compound 1 also displayed superior Cyclophilin D inhibition, with at least a 50 fold improvement relative to all other analogues tested.

In an embodiment, of the compound, method or use as mentioned herein above, the organ can be kidney, pancreas, liver, heart, lungs or intestines. In the case of severed body parts, the body parts may be limbs, hands, feet, fingers or toes.

In an embodiment, of the compound, method or use as mentioned herein above, the dose of the compound is 0.1 to 10 mg/kg; and optionally is 1 to 3 mg/kg. In the case where an organ or body part is bathed in a fluid containing the compound of the invention, or this fluid is being recirculated, the concentration of the compound may be higher or lower as required by need.

In an embodiment, of the compound, method or use as mentioned herein above, the compound is formulated in Cremophor (polyethoxylated castor oil)/saline/DMSO (dimethyl sulfoxide).

According to one aspect of the disclosure, there is provided a method of preparing a compound of Formula 1, the method comprising a product forming reaction, the reaction comprising copper triflate and an amino alcohol of Formula 2: 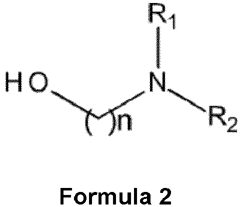
wherein n is 2-5, and
R₁ and R₂ are independently selected from H or C₁-C₄ alkyl, wherein R₁ and R₂ may be joined together to form a C₃-C₅ heteroalkyl ring.

Surprisingly, it has been found that the compound of the invention is made readily available by the above mentioned method. For example, many failed attempts were made by the present applicant to replicate the methodology disclosed in US 6,583,265, and in the end the approach in US 6,583,265 was abandoned, being unviable.

In an embodiment, the reaction comprises a drying agent and/or is performed under substantially anhydrous conditions. Optionally the drying agent is molecular sieves. Optionally the molecular sieves are 3 A molecular sieves. In an embodiment, the amino alcohol is N,N-dimethylaminoethanol. This amino alcohol gives Compound 1. In an embodiment, the amino alcohol reacts with a cyclosporine precursor compound comprising a labile group, wherein the labile group is lost in the reaction. Optionally, the labile group is bonded to the precursor compound by a -S- bond. Further optionally the labile group is a thiopyridyl group or a mercaptobenzothiazole-2-ylthio group.

### Brief description of the drawings

Figure 1 shows the inhibitory and/or protective effect of Compound 1, and of comparative compound CsA, on induced Acute Kidney Injury in rats, by measuring blood serum Creatinine concentrations.
Figure 2 shows the inhibitory and/or protective effect of Compound 1, and of comparative compound CsA, on induced Acute Kidney Injury in rats by measuring Blood Urea Nitrogen (BUN) concentrations.
Figure 3 shows the inhibitory and/or protective effect of Compound 1 against LPS induced Acute Kidney injury.
Figure 4 shows the effects of Compound 1 on kidney function. Lower creatinine and blood urea nitrogen levels for animals treated with Compound 1 are consistent with reduced levels of damage to the kidney.

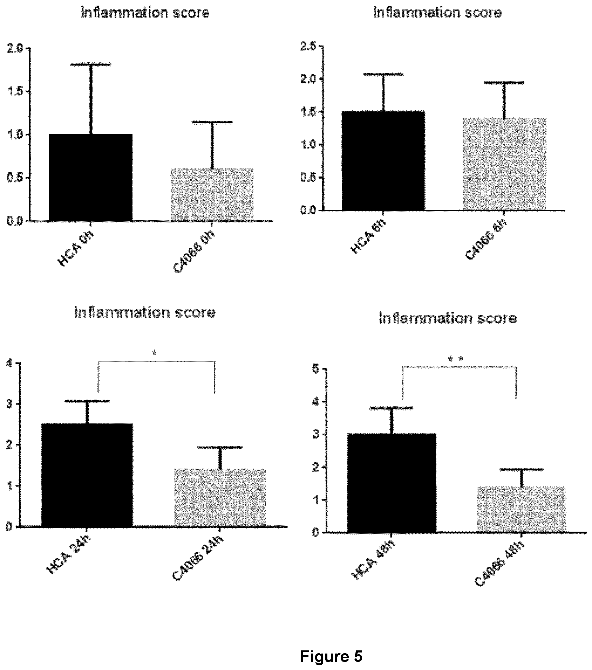

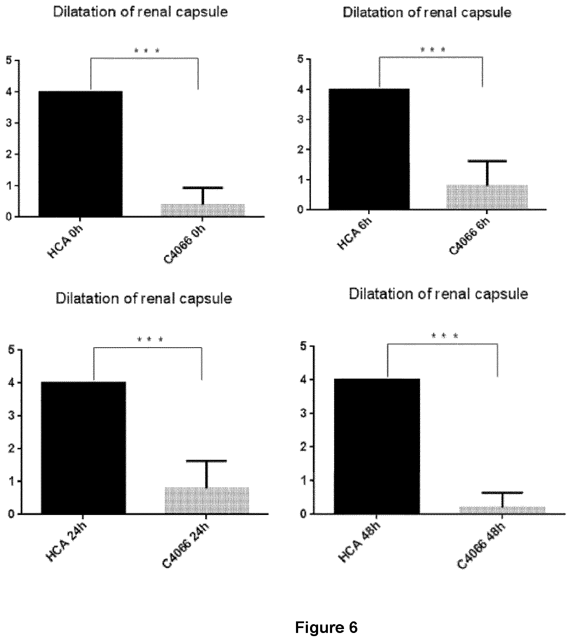

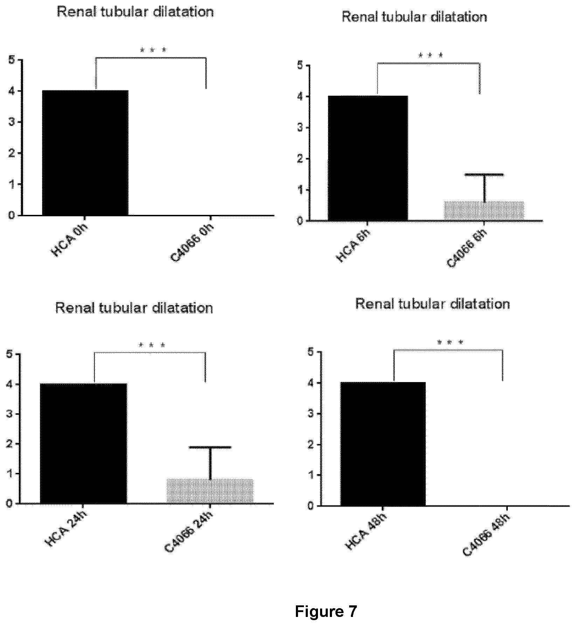

Figures 5-7 show data showing kidney preservation ex-vivo after removal. 4 kidneys for control, without protectant compound i.v. (intravenous injection) dose before kidney perfusion, 5 kidneys received 5 mg/kg C4066 (compound 1) i.v. dose 1 hr before kidney perfusion. Data shows the average score across the studied kidneys at times after removal at 0, 6, 24 and 48 h. HE score criteria: according to the degree of inflammation from mild to severe, followed by semi-quantitative scoring, for very small or no lesion negative "-" 0; mild or small "+" 1; moderate or medium size "+" 2; severe or large "++" 3; extremely severe or large "+++" 4. Figure 5 shows the Inflammation score, Figure 6 the Dilation of the renal capsule and Figure 7 the Renal tubular dilation.

Chinese key to drawings:
Fig. 1: Creatinine: creatinine.

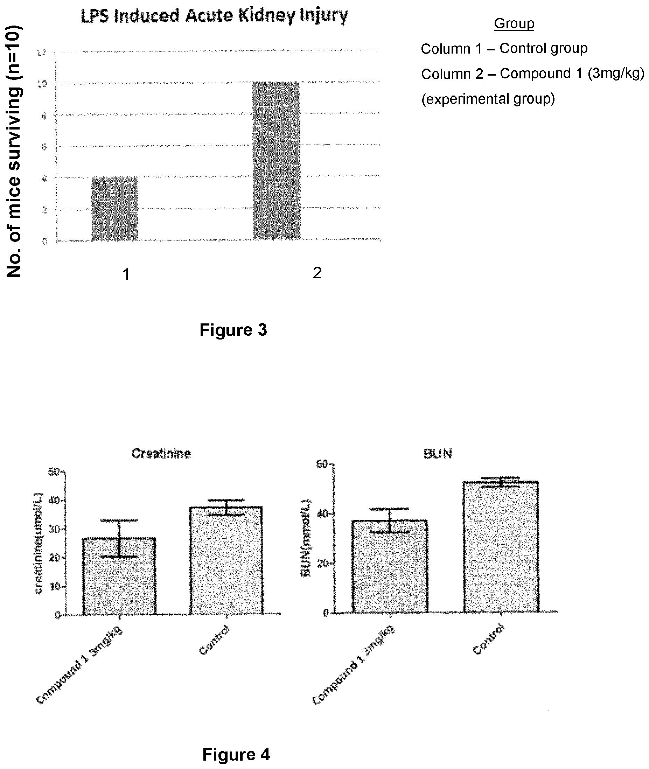

Fig. 3: LPS induced acute kidney injury: LPS induced acute kidney injury
Fig. 4: Control: control; Compound: compound; Creatinine: creatinine.
Fig. 5: Inflammation score: inflammation score.
Fig. 6: Dilatation of renal capsule: dilatation of renal capsule.

### Detailed Description

The invention will now be illustrated by the following examples.

### Experimental methods and results

The skilled person will recognise that compounds of Formula 1 may be prepared in a variety of ways. The route below is merely one example of a way that could be employed for the synthesis of Compound 1. That said, the route used to prepare Compound 1 in US 6,583,265 was not effective. Many attempts were made to replicate the methodology in US 6,583,265 without great success. Without being bound by theory, it is believed that the dimethyl amino group (being basic) reacted preferentially with the acid catalyst. The acid catalyst was thereby prevented from activating the loss of the leaving group, inhibiting the progress of the reaction.

Compounds of general formula 1 can be conveniently prepared using several pathways. In one instance (Scheme 1), reaction of compound 2, in which R is lower alkyl, with a carbonyl compound and a reducing agent can perform a reductive amination procedure to give the desired compounds. Preferably the carbonyl compound is a lower alkyl aldehyde or ketone and the reducing agent is a metal borohydride. More preferably the aldehyde is formaldehyde, acetaldehyde or propionaldehyde and the ketone is acetone or 2-butanone and the like. Preferably the reducing agent is sodium triacetoxyborohydride or sodium cyanoborohydride.

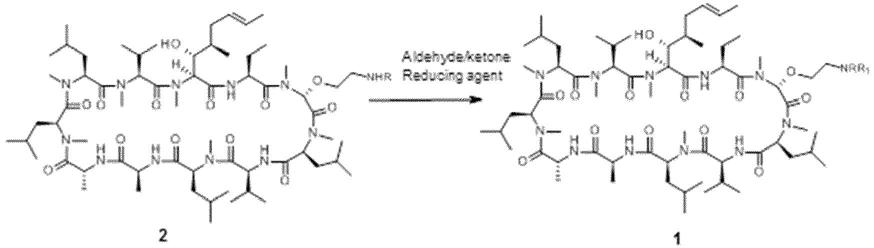

The amine compound 2 can be conveniently prepared from a suitably protected ethanolamine compound such as 3, wherein R is hydrogen or lower alkyl, by treating said compound with conditions known to remove the protecting group and yielding the free amine compound. Suitable protecting groups that can be removed in the presence of other functional groups in the molecule include tert-butoxycarbonyl (BOC), 9-fluorenylmethyloxycarbonyl (FMOC) and the like. Preferably the protecting group is tert-butoxycarbonyl (BOC) and the conditions employed for removal of the BOC group involve treatment with acid, such as trifluoroacetic acid.

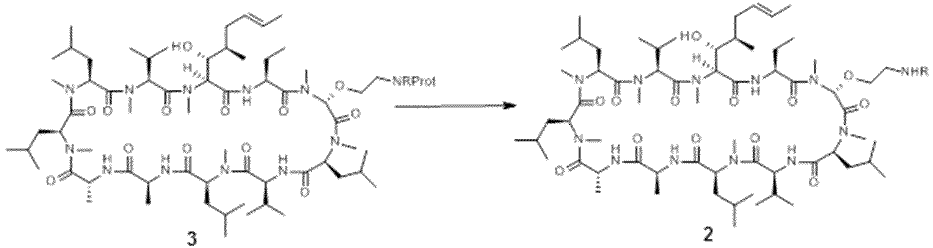

Step 1: Preparation of [2'-(2-Thiopyridyl)-Sar]³-cyclosporine A

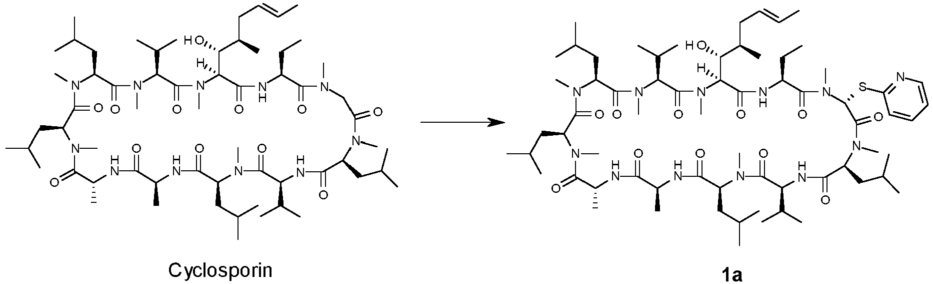

### [2'-(2-Thiopyridyl)-Sar]³-cyclosporine A (1a)

To a dry 1L flask was added cyclosporine A (20 g, 16.6 mmol), anhydrous lithium chloride (21.1 g, 499 mmol) and dry THF (500 mL), the flask was then flushed with argon and the mixture was cooled to -45 °C. In a separate flask, diisopropylamine (13.5 g, 133 mmol) was dissolved in dry THF (120 mL) and cooled to -78 °C. To this flask was added n-butyllithium (53.2 mL of a 2.5 M solution, 133 mmol) and the resulting solution was stirred at -78 °C for 20 min. Using a canula, the solution of lithium diisopropylamide was transferred to the solution of cyclosporine and the resulting mixture was stirred at -45 °C for 90 min. A solution of 2-pyridyldisulfide (11 g, 49.9 mmol) in dry THF (20 mL) was added dropwise and the resulting mixture was allowed to warm to room temperature overnight. The reaction was quenched by the careful addition of saturated NaCl solution (200 mL) and the resulting organic layer was separated. The aqueous layer was extracted with ethyl acetate (3 × 100 mL) and the combined organic fractions were washed with 3N NaOH (2 × 100 mL), saturated NH₄Cl (100 mL) and saturated NaCl (100 mL) followed by drying over anhydrous Na₂SO₄ and evaporation. The title compound was isolated by silica gel chromatography as a solid, 7.18g. ¹H NMR (400 MHz, CHLOROFORM-d) d 8.45 (ddd, J=0.88, 1.73, 4.90 Hz, 1H), 7.98 (d, J=9.66 Hz, 1H), 7.65-7.73 (m, 1H), 7.59 (dt, J=1.85, 7.71 Hz, 1H), 7.51 (ddd, J=0.76, 1.68, 6.44 Hz, 0H), 7.45 (d, *J*=8.54 Hz, 1H), 7.35 (ddd, J=1.73, 6.97, 8.77 Hz, 0H), 7.25 (s, 0H), 7.17 (d, J=7.96 Hz, 1H), 7.09-7.15 (m, 2H), 6.72 (dt, J=1.17, 6.71 Hz, 0H), 5.70 (dd, J=4.29, 10.88 Hz, 1H), 5.50 (d, J=6.39 Hz, 1H), 5.32-5.38 (m, 1H), 5.28 (dd, J=3.88, 11.74 Hz, 1H), 5.13 (d, J=10.88 Hz, 1H), 4.97-5.11 (m, 2H), 4.84 (dq, J=7.03, 7.24 Hz, 1H), 4.69 (t, J=9.15 Hz, 1H), 4.54 (quin, J=7.31 Hz, 1H), 4.13 (q, J=7.16 Hz, 0H), 3.81 (dt, J=1.00, 5.75 Hz, 1H), 3.59-3.72 (m, 1H), 3.50 (s, 2H), 3.38 (s, 2H), 3.26 (s, 2H), 3.13 (s, 5H), 2.70 (d, J=1.07 Hz, 5H), 2.34-2.54 (m, 1H), 1.92-2.23 (m, 4H), 1.55-1.85 (m, 11H), 1.19-1.54 (m, 11H), 1.12 (d, J=6.54 Hz, 2H), 0.78-1.07 (m, 30H), 0.73 (d, 3H).

### Step 2: Preparation of [2'-(2-Dimethylaminoethoxy)-Sar]³-cyclosporine A (Compound 1)

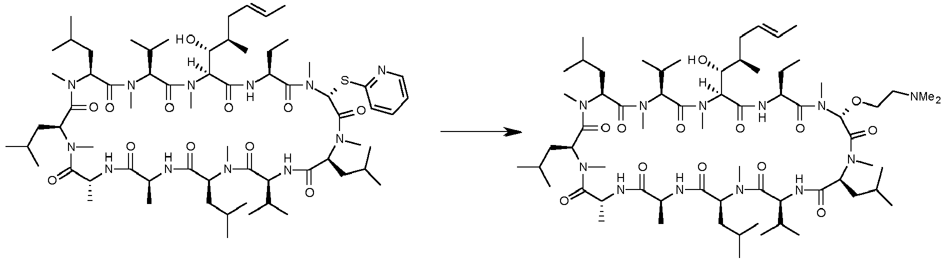

### [2'-(2-Dimethylaminoethoxy)-Sar]³-cyclosporine A (1)

Copper triflate (0.291 g, 0.8 mmol) and 3 angstrom molecular sieves were added to a flask, dry THF (3 mL) was added and the flask was flushed with argon. In a separate flask, a mixture of [2'-(2-thiopyridyl)-Sar]³-cyclosporine A (1a) (0.293 g, 0.223 mmol), dimethylaminoethanol (0.086 g, 0.96 mmol) and 3 A molecular sieves in dry THF (2 mL) was stirred for 30 minutes and then added to the copper triflate solution. The reaction was allowed to stir at room temperature overnight. A saturated solution of NaHCO₃ (10 mL) was added and the mixture was filtered through celite. The celite was washed with ethyl acetate (3 × 25 mL) and added to the filtrate. The organic layer was separated; the aqueous layer was extracted with EtOAc (2 × 25 mL) and the combined organic fractions were dried over anhydrous Na₂SO₄ and evaporated. Purification of the crude extract on silica gel afforded the title compound, 86.4 mg. ¹H NMR (400 MHz, CHLOROFORM-d) d 7.92 (d, J=9.61 Hz, 1H), 7.75 (d, J=7.32 Hz, 1H), 7.22 (d, J=8.15 Hz), 7.15 (d, J=7.86 Hz,), 6.01 (s, 1H), 5.70 (dd, J=4.22, 10.86 Hz, 1H), 5.46 (d, J=6.10 Hz, 1H), 5.35 (q, J=4.77 Hz, 1H), 5.27 (dd, J=4.15, 11.42 Hz, 1H), 5.14 (d, J=10.83 Hz, 1H), 5.05-5.11 (m, 1H), 4.94-5.04 (m, 1H), 4.77-4.90 (m, 1H), 4.73 (s), 4.66 (t, J=8.83 Hz, 1H), 4.46-4.57 (m, 1H), 3.71-3.81 (m, 1H), 3.58-3.67 (m, J=5.15, 5.64, 5.64, 5.83 Hz, 1H), 3.53-3.58 (m, 1H), 3.51 (s, 2H), 3.24 (s, 2H), 3.20 (s, 2H), 3.13 (d, J=2.10 Hz, 3H), 2.71 (d, J=6.54 Hz, 3H), 2.49-2.67 (m, 2H), 2.33-2.46 (m, 1H), 2.27 (s, 4H), 1.88-2.20 (m, 4H), 1.74 (d, J=0.29 Hz, 6H), 1.57-1.68 (m, 5H), 1.38-1.52 (m, 2H), 1.35 (d, J=7.27 Hz, 3H), 1.26 (d, *J*=2.88 Hz, 4H), 0.77-1.12 (m, 30H), 0.70 (d, 2H).

The skilled person will for example appreciate that analogues of Compound 1 can be made by using different amino alcohol reagents. For example, the number of carbon atoms between the alcohol and amine group could be increased or decreased (examples of linking groups include: methylene, ethylene, propylene, butylene, pentalene, and may include branched versions thereof, such as iso-propylene, sec-butylene, tert-butylene, 2-methylbutylene, 2,2-dimethylpropylene). Alternatively, or in addition, the N-amino substituents on the amino alcohol could also be changed to give further analogues of Compound 1 (examples of N-amino substituents include: methyl, ethyl, propyl, isopropyl, n-butyl, sec-butyl, tert-butyl, butyl).

### Preparation of [2'-(2-N-Boc-aminoethoxy)-Sar]³-cyclosporine A

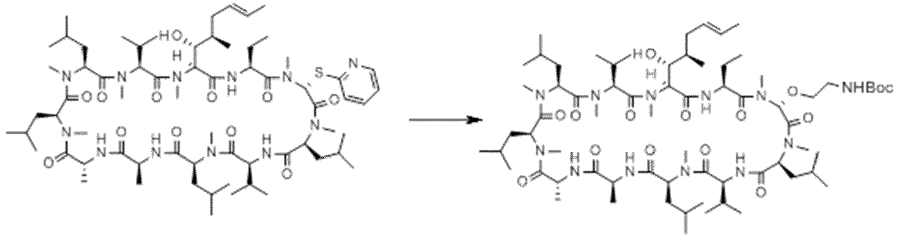

Copper triflate (4.95 g, 13.7 mmol) and 3 A molecular sieves were suspended in anhydrous THF (50 mL) and stirred under argon for 30 min. A solution of [2'-(2-thiopyridyl)-Sar]³-cyclosporine A (1a) (5.0 g, 3.82 mmol) and N-Boc-ethanolamine (2.64 g, 16.4 mmol) in anhydrous THF (10 mL) was dried over 3 A molecular sieves for 30 min and then added to the copper triflate suspension. The resulting mixture was stirred at room temperature overnight. Saturated NaHCO₃ (2 × 50 mL) was added and the mixture was filtered through Celite. The Celite was washed with EtOAc (4 × 100 mL) and the organic layer was separated. The aqueous phase was extracted with EtOAc (2 × 50 mL) and the combined organic fractions were washed with saturated NaCl (50 mL), dried over anhydrous Na₂SO₄ and evaporated. The crude product was purified on silica to yield the title compound, 4.18 g. ¹H NMR (400 MHz, CHLOROFORM-d) d ppm 0.72 (ddd, 2 H) 0.91 (m, 31 H) 1.32 (m, 8 H) 1.48 (dddd, J=3.95, 3.07, 2.23, 0.95 Hz, 2 H) 1.69 (m, 10 H) 2.10 (m, 4 H) 2.39 (m, 1 H) 2.70 (m, 4 H) 2.95 (m, 2 H) 3.12 (d, J=7.42 Hz, 4 H) 3.17 (d, J=9.37 Hz, 1 H) 3.20 (s, 2 H) 3.25 (s, 2 H) 3.29 (m, J=6.69, 3.02, 1.45, 0.76, 0.63 Hz, 1 H) 3.41 (m, 1 H) 3.51 (s, 2 H) 3.61 (m, 1 H) 3.75 (dddd, J=7.73, 1.54, 1.02, 0.73 Hz, 1 H) 4.13 (q, J=7.11 Hz, 1 H) 4.50 (m, 1 H) 4.65 (dd, J=18.06, 0.44 Hz, 1 H) 4.98 (m, 4 H) 5.30 (m, 2 H) 5.47 (m, 1 H) 5.70 (m, 1 H) 5.93 (d, J=0.34 Hz) 7.21 (m, 1 H) 7.71 (m) 8.03 (m).

### Preparation of [2'-(2-aminoethoxy)-Sar]³-cyclosporine A

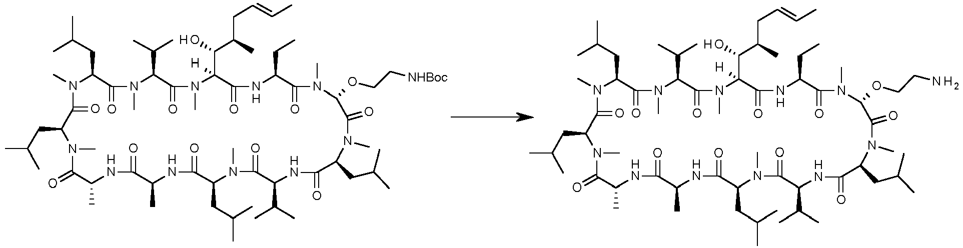

A solution of [2'-(2-N-Boc-aminoethoxy)-Sar]³-cyclosporine A (3) (3.0 g, 2.2 mmol) in dry CH₂Cl₂ (30 mL) was cooled to 0 °C and trifluoroacetic acid (6.54 mL, 10.03 g, 88 mmol) was added dropwise and the mixture was stirred for 30 min. The solvent was evaporated and the crude product was purified on silica to deliver the title compound, 1.99 g.

### Preparation of [2'-(2-Dimethylaminoethoxy)-Sar]³-cyclosporine A (2)

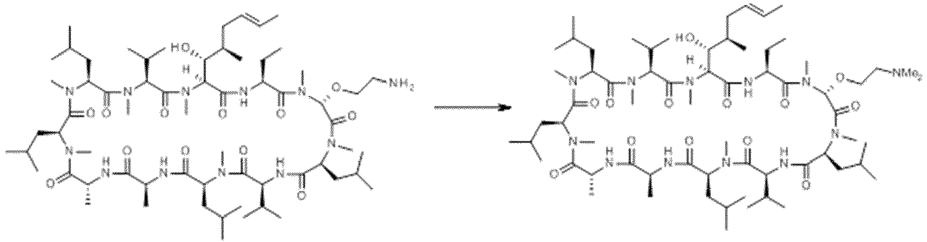

[2'-(2-Aminoethoxy)-Sar]³-cyclosporine A (0.273 g, 0.216 mmol) was dissolved in CH₂Cl₂ (5 mL) and formaldehyde (37% aqueous sol., 0.048 mL, 0.69 mmol) was added followed by NaB(OAc)₃H (0.138 g, 0.649 mmol) and the reaction was allowed to stir at about room temperature for 18h. The reaction mixture was filtered through a small pad of silica gel which was washed with 90:9:1 CH₂Cl_{2:}MeOH:conc.NH₄OH (5 × 100 mL). The solvent was evaporated and the product was isolated by chromatography on silica gel to afford the title compound, 0.214 g.

### Cyclophilin inhibition binding measurements

The cyclophilin inhibition binding activity of compounds disclosed herein was determined using a competitive ELISA adapted from the methods described by Quesniaux et al. (Eur. J Immunol., 1987, 17:1359-1365). Activated ester of succinyl spacers bound to D-Lys⁸-cylosporine A (D-Lys⁸-Cs) are coupled to bovine serum albumin (BSA) through D-lysyl residue in position 8. BSA is dissolved in 0.1 M borate buffer, pH 9.0 (4 mg in 1.4 ml). A hundredfold molar excess of D-Lys⁸-Cs dissolved in dimethyl formamide (0.6 ml) is added dropwise to the BSA under vigorous stirring. The coupling reaction is performed for 2 to 3 hours at room temperature under mild stirring and the conjugate obtained is extensively dialyzed against phosphate-buffered saline (PBS, pH 7.4). After acetone precipitation of an aliquot of the conjugated protein, no covalently bound D-Lys⁸-Cs remains in the acetone solution and the extent of cyclosporine covalent binding is then calculated.

Microtiter Plates are coated with D-Lys⁸-Cs-BSA conjugate (2 µg/ml in PBS for 24 hours at 4°C). Titration Plates are washed with Tween^{®}/PBS and with PBS alone. To block nonspecific binding, 2% BSA/PBS (pH 7.4) is added to the wells and allowed to incubate for 2 hours at 37°C. A five-fold dilution series of the compound to be tested is made in ethanol in a separate microtiter plate. The starting concentration is 0.1 mg/mL for assays with human recombinant cyclophilin. 198 µL of 0.1 µg/mL cyclophilin solution is added to the microtiter plate immediately followed by 2 µL of diluted cyclosporine A (used as a reference compound) or the compound of the invention. The reaction between coated BSA-Cs conjugate, free cyclosporine A and cyclophilin is allowed to equilibrate overnight at 4°C. Cyclophilin is detected with anti-cyclophilin rabbit serum diluted in PBS containing 1% BSA and incubates overnight at 4°C. Titration Plates are washed as described above. Bound rabbit antibodies are then detected by goat anti-rabbit IgG conjugated to alkaline phosphatase and diluted in 1% BSA-PBS and allowed to incubate for 2 hours at 37°C. Titration Plates are washed as described above. After incubation with 4-nitrophenyl phosphate (1 g/l in diethanolamine buffer, pH 9.8) for 1 to 2 hours at 37°C, the enzymatic reaction is measured spectrophotometrically at 405 nm using a spectrophotometer. The results are expressed as an EC₅₀, which is the concentration of the compound of the invention required to achieve 50% inhibition. Compound 1 had EC₅₀ values of less than 100nM against cyclophilins A, B and D.

### PPlase Inhibition

The assay was performed using an Agilent 8453 spectrophotometer essentially as described as the 'uncoupled assay' by Janowski *et al.* {Jankowski et al. Anal. Biochem. (1997), 252:299-307}. Assay buffer consisting of 35 mM HEPES pH 7.8 and 50 µM DTT was cooled to 10 °C (with stirring) in a precision glass cuvette and inhibitor was added from a 100% DMSO stock solution. A blank spectrum was obtained and then purified His tagged recombinant human cyclophilin enzyme (f/c 2 nM) and tetra peptide substrate (Suc-Ala-Ala-Pro-Phe-para-nitroanilide dissolved in a solution of 0.5 M LiCI in trifluoroethanol (Bachem, f/c 60 µM)) were added and the change in absorbance measured over 5 min at 330 nM. A first order rate equation was fitted to the absorbance data to obtain a rate constant (first 10 to 15 s were eliminated due to mixing). The catalytic rate was calculated from the enzymatic rate minus the background rate. The *K*ᵢ for an inhibitor was obtained from the rate constant plotted against the inhibitor concentration.

### Mitochondrial Permeability Transition

Mitochondrial Permeability Transition (MPT) is determined by measuring swelling of the mitochondria induced by Ca²⁺. The procedure is adapted from the method described by Blattner et al., 2001, Analytical Biochem, 295:220. Mitochondria are prepared from rat livers, which have been perfused with phosphate-buffered saline (PBS) to remove blood, using standard methods that utilize gentle homogenization in sucrose based buffer and then differential centrifugation to first remove cellular debris and then to precipitate the mitochondria. Swelling is induced by 150 micro molar Ca²⁺ (added from a concentrated solution of CaCl₂) and is monitored by measuring the scattering at 535-540 nm. Representative compounds are added 5 minutes before swelling is induced. EC₅₀ is determined by comparing swelling with and without the compounds disclosed herein. Compound 1 inhibited mitochondrial swelling with an EC₅₀ of less than 0.2 µM.

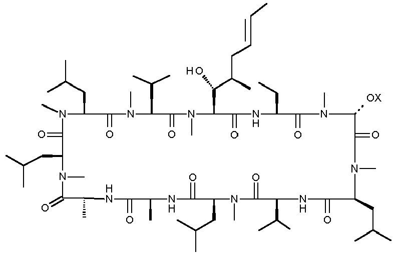

**Table 1 - Measurement results for Cyclophilin A inhibition, Cyclophilin D inhibition and Mitochondrial Permeability Transition (MPT).**

| **Entry** | **X** | **CypA EC₅₀ (nM)** | **CypD EC₅₀ (nM)** | **MPT (µM)** |
|---|---|---|---|---|
| **1** | 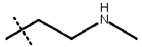 | 61 | 2170 | 10 |
| **2** | 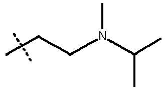 | 202 | 3550 | 7.6 |
| **3** | 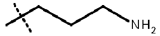 | 14 | ND | 2.69 |
| **4** *Compound 1* | 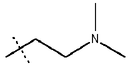 | 60 | 24 | 0.1 |
| **5** | 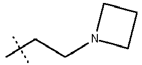 | 66 | ND | 10 |
| **6** | 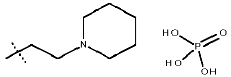 | 118 | 2500 | 7.5 |
| **7** | 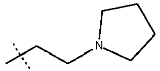 | 12 | 1200 | 10 |

The results displayed in Table 1 demonstrate the unexpectedly high Cyclophilin D inhibition and MPT of Compound 1 (entry 4) relative to similar analogues (entries 1-3 and 5-7). A 100 fold improvement in MPT was observed relative to three other compounds (entries 1, 5 and 7) and an over 25 fold improvement was observed relative to the next best performing analogue (entry 3). Compound 1 also displayed superior Cyclophilin D inhibition, with at least a 50 fold improvement relative to all other analogues tested.

### Protective effects of Compound 1 in animal models of organ damage

### Acute Kidney Injury induced by renal ischemia-reperfusion injury

Compound 1 and Cyclosporin A formulations were prepared by mixing these compounds with Cremophor/saline/DMSO.

Sprague-Dawley rats were divided into six groups: Group (i) the sham group, dosed with Cremophor/saline/DMSO with no active component; Group (ii) the control group, dosed with

Cremophor/saline/DMSO with no active component; Group (iii) dosed with Compound 1 (3 mg/kg); Group (iv) dosed with CsA (3 mg/kg); Group (v) dosed with Compound 1 (10 mg/kg); Group (vi) dosed with CsA (10 mg/kg). With the exception of Group (i), i.e. the 'sham group', renal Ischemia-Reperfusion Induced Acute Kidney Injury (AKI) was induced in the rats by ligation of bilateral renal arteries for 30 min and then release of ligation.

Animals in the control and treatment groups were administered intraperitoneal injections three times (1h before ligation, 4h and 8h after ligation). Blood was taken from the animals 24 hours after the ligation/release procedure and analyzed for serum **Creatinine** and **Blood Urea Nitrogen** (BUN) concentrations, as a measure of kidney injury.

The results of those experiments are shown below, and graphically in Figures 1 and 2.

**Table 2 - Measurement results for serum Creatinine and BUN concentrations of Groups (i) to (vi)**

| | **Creatinine (umol/L)** | **Blood Urea Nitrogen (mmol/L)** |
|---|---|---|
| Group (i) | 25 | 5 |
| Group (ii) | 195 | 38 |
| Group (iii) | 60 | 14 |
| Group (iv) | 115 | 22 |
| Group (v) | 250 | 40 |
| Group (vi) | 290 | 42 |

### Discussion of results

In Figure 1 the blood serum Creatinine concentration is indicative of kidney damage. The 'sham group' are rats without induced AKI. The 'control group' represents rats with induced AKI, and which are untreated. Therefore, it can be seen that induced AKI results in increased levels of blood serum Creatinine from 25 µmol/ml (Group i) to 195 µmol/ml group (Group ii). Treating rats with induced AKI with 3 mg/kg of CsA (Group iv) results in the Creatinine levels dropping from 195 µmol/ml to 115 µmol/ml as compared to Group ii. Therefore, it is understood that CsA is acting to prevent the ischaemia-reperfusion injury.

Surprisingly, when rats with induced AKI are treated with 3 mg/kg of Compound 1 (Group iii), this gives a very marked reduction in Creatinine levels, dropping from 195 umol/ml to 60 umol/ml (as compared to Group ii), which is approaching the Creatinine levels seen in the 'sham group' (Group i), i.e. rats with no induced AKI. When the doses of Compound 1 and CsA are increased from 3 mg/ml (Groups iii and iv) to 10 mg/ml (Groups v and vi), it appears that the benefit of the CsA and Compound 1 are reduced, with Compound 1 still performing better than CsA.

In Figure 2 the Blood Urea Nitrogen (BUN) concentration is indicative of kidney damage.

Figure 2 follows the same trend as seen in Figure 1. That is, 3 mg/kg of CsA results in a drop in BUN levels (Group iv compared to Group ii), whereas 3 mg/kg of Compound 1 shows a very marked reduction in BUN levels (Group iii compared to Group ii), getting towards the BUN level seen in the 'sham group' (Group i). Increasing the concentration of Compound 1 and CsA from 3 mg/kg (Groups iii and v) to 10 mg/kg (Groups v and vi) proves to be less effective. This result supports the result seen in Figure 1.

### Acute Kidney Injury induced by lipopolysaccharide (LPS) challenge

LPS induced Acute Kidney Injury (AKI) was induced in mice (C57) by intraperitoneal injection of LPS (15 mg/kg). Twenty mice were randomly divided into two groups. Animals in the control group received vehicle (Cremophor/saline/DMSO) and the treatment group received Compound 1 (3 mg/kg in Cremophor/saline/DMSO) each dosed intraperitoneally. The animals were dosed with vehicle or Compound 1 three times (1 h before LPS injection and 4h and 8h after LPS injection) and blood was taken from the animals 12 h after LPS injection. The activity of the compound was determined by increased survival rate (Figure 3) and by evaluation of markers of kidney function (Figure 4).

| | **Survival** | **Creatinine (umol/L)** | **Blood Urea Nitrogen (mmol/L)** |
|---|---|---|---|
| Control | 40% | 37 | 52 |
| Compound 1 | 100% | 26 | 38 |

### Discussion of results

In Figure 3 the protective effects of Compound 1 are presented in terms of animal survival. At a dose level of 3 mpk, Compound 1 administration resulted in survival of all animals in the group compared to only 4 out of 10 animals in the control group which did not receive Compound 1.

Figure 4 shows the effects of Compound 1 on kidney function in this experiment. Lower creatinine and blood urea nitrogen levels for animals treated with Compound 1 are consistent with reduced levels of damage to the kidney.

*Organ protection during transplantation by administration to an organ donor.*

The protective effect of Compound 1 toward an organ subjected to conditions of transplantation was exemplified using pig kidneys. In the experiment conducted, a single dose of Compound 1 was administered to the pig at 5 mg/kg via intravenous delivery 1 hr before kidney resection. The kidney was resected, perfused with standard hypertonic citrate adenine (HCA) preservation fluid and then preserved in HCA solution at low temperature (0°C-4°C). The organ was monitored to record damage by histologic evaluation and measurement of inflammatory markers over several time points after the resection procedure:
O Zero point
O 6h
O 24h
O 48h.

Data is shown in Figures 5-7.

Histologic evaluation was made following hemotoxalyn and eosin (HE) staining using score criteria according to the degree of inflammation. A semi-quantitative scoring system, "0 to 4" was employed in which very small or no lesion is assigned "0"; mild or small is assigned "1"; moderate is assigned "2"; severe is assigned "3"; extremely severe is assigned "4".

The experiment was conducted with a total of 9 pig kidneys in which 4 kidneys were used as controls, without protectant compound, and 5 kidneys received 5 mg/kg Compound 1 i.v. dose 1 hr before kidney resection. Data shows the average across the studied kidneys at times after removal.
Figure 5 shows the results of an averaged inflammation score;
Figure 6 shows the effects on dilation of the renal capsule and
Figure 7 shows the effects on renal tubular dilation.

### Discussion of results

In overall summary, Compound 1 was found to be surprisingly efficacious in the treatment or prevention of ischaemia-reperfusion injury, in particular at lower concentration levels. The compound is also particularly efficacious administered to an organ donor prior to removal of an organ (for subsequent implantation to a recipient). Figures 5 to 7 show that an organ can be preserved ex-vivo by administering the compound to a donor prior to organ removal.

## Claims

1. Use of a mitochondrial protectant compound for preservation of an organ, wherein the compound is administered to an organ donor in order to protect the organ prior to removal of said organ from said donor.

2. Use according to claim 1 wherein the compound is a Cyclophilin D inhibitor.

3. Use according to claim 2 wherein the compound is a compound of Formula 1: 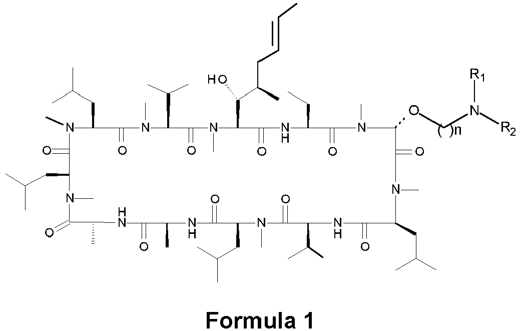 or a salt thereof;
wherein n is 2-5, and
R₁ and R₂ are independently selected from H or C₁-C₄ alkyl, wherein R₁ and R₂ may be joined together to form a C₃-C₅ heteroalkyl ring.

4. Compound according to claim 3 wherein the compound is Compound 1: 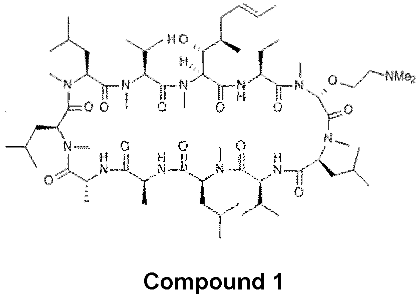 or a salt thereof.

5. The use according to any one of claims 1 to 4 wherein the organ is a kidney.

6. A method of preserving a kidney from a kidney donor comprising administering a compound to said donor prior to removal of said kidney from said donor, wherein the compound is Compound 1: 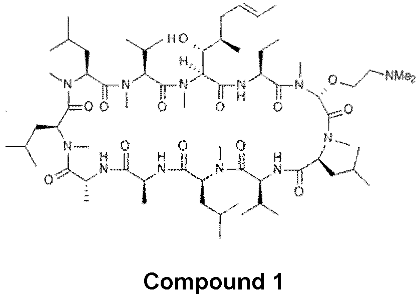 or a salt thereof.

7. The method or use according to any one of claims 1 to 6 wherein the donor is a live donor.

8. The use of a mitochondrial protectant compound for the manufacture of a medicament for administration to a live donor for the preservation of a kidney, wherein the compound is Compound 1: 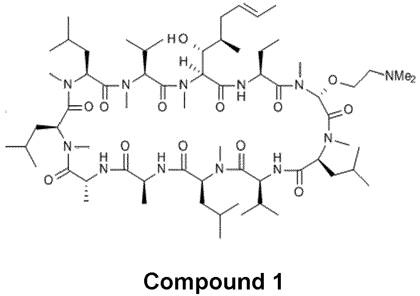 or a salt thereof.

9. A method or use according to any one of claims 1 to 7, wherein the dose of the compound is 0.1 to 10 mg/kg.

10. A method or use according to claim 9, wherein the dose of the compound is 1 to 3 mg/kg.
